# EUROPEAN PATENT APPLICATION

(11) **EP 1 550 666 A1**
(43) Date of publication of application: **06.07.2005**
(21) Application number: 05000058.7
(22) Date of filing: 04.01.2005
(51) Int. Cl.: C07H 3/04, C13F 3/00, C12P 19/18

(54) **Method for preparing crystalline isomaltulose and hydrogenated isomaltulose**

(30) Priority: 05.01.2004 JP 2004000469
(71) Applicant: KABUSHIKI KAISHA UENO SEIYAKU OYO KENKYUJO, Osaka-shi, Osaka-fu (JP)
(72) Inventor: Honda, Junya, Nishinomiya-shi, Hyogo-ken (JP); Kuriyama, Yoshiaki, Tsukuba-shi,Ibaraki-ken (JP); Furukawa, Yojiro, Itami-shi,Hyogo-ken (JP); Ueno, Takao, Kobe-shi, Hyogo-ken (JP)
(74) Representative: Albrecht, Thomas, Dr.

(57) **Abstract**

Provided is a method for manufacturing crystalline isomaltulose from sucrose, comprising the steps of: 1) contacting an α-glucosyltransferase enzyme to aqueous sucrose solution or slurry under the condition wherein the α-glucosyltransferase enzyme is active; in which said condition is maintained after the concentration of isomaltulose in the reaction mixture reaches the point at which crystals are formed, and 2) separating the reaction mixture into crystalline isomaltulose and remaining syrup. According to the present invention, enzymatic conversion of sucrose and crystallization of isomaltulose are carried out simultaneously in a same reaction vessel. In addition, the enzyme can be used repeatedly.

## Description

### Technical Field

The present invention relates to a method for preparing crystalline isomaltulose from sucrose. The present invention also relates to a method for preparing hydrogenated isomaltulose.

### Background Art

Isomaltulose, which is also called as palationose, is a disaccharide of glucose and fructose of which glucosidic linkage is α-1,6. Isomaltulose is manufactured by an enzymatic conversion of sucrose, whereby the 1,2-glycosidic linkage between glucose and fructose is rearranged to a 1,6-glycosidic linkage. It is used in a wide variety of food products as an alternative for sugar or sweeteners. Isomaltulose is less sweet, gentler on teeth and induces less insulin secretion as compared to sucrose.

Hydrogenated isomaltulose, which is also called as isomalt and is known as "Palatinit®", is obtained by hydrogenating isomaltulose. Hydrogenated isomaltulose is known to have similar preferable properties as isomaltulose. In addition to the properties of isomaltulose, isomalt is much less hygroscopic, provides very low caloric value and is excellent in heat resistance. Because of those superior properties, isomalt is used in more and more food products than isomaltulose.

Alpha glucosyltransferases, which is used for the conversion of sucrose into isomaltulose, are produced by various microorganisms such as of the genus *Serratia, Klebsiella, Pseudomonas, Protaminobacter, Elvinia* and *Agrobacterium.*

Industrial process for manufacturing isomaltulose comprises enzymatic conversion of sucrose with α-glucosyltransferase under a condition wherein temperature, pH and the like are suitable for the enzymatic reaction. According to this enzymatic reaction process, a by product trehalulose is produced in the reaction mixture in addition to isomaltulose. In order to isolate isomaltulose and to give the product with high purity, thus obtained mixed solution of isomaltulose and trehalulose is then subjected to the crystallization. In the process, crystallized isomaltulose is isolated from the mixed syrup comprising isomaltulose, trehalulose and the enzyme.

The crystallization process comprises the steps of concentrating the syrup and/or cooling the same. That is, equipments being able to effectuate heating and depressuring at the same time and/or that to reduce the temperature in a constant manner are required. In addition, large amount of energy is required for distilling off the water content or cooling down the mixture, which causes higher running cost.

In the enzymatic reaction step, the most convenient way to contact the enzyme with the substrate is adding cell-free enzyme extract into the starting aqueous sucrose solution. However, since the enzymes are very expensive, some efforts for reducing total amount of α-glucosyltransferase enzyme used in throughout the process have been made. For example, processes such as using immobilized enzyme or enzyme producing cells, or having the step to collect the enzyme after the reaction with semipermeable membrane are proposed so far. In thus proposed processes, the enzyme must be removed from the reaction mixture before subjecting to the crystallization step. It can be achieved by, for example, carrying out the enzymatic reaction with a column filled with carriers on which the enzyme is immobilized, or separating the enzyme from the reaction mixture after the enzymatic reaction is completed with semipermeable membranes. In those processes, enzymatic reaction and crystallization are carried out separately, and the process requires to use immobilized enzyme and/or to have means for separating the enzyme from the reaction mixture.

In one typical conventional process, for example, 40wt% aqueous sucrose solution is converted using a column filled with carriers on which the enzymes are immobilized under the condition of pH 5.5 at 50°C. The aqueous sucrose solution is loaded at a flow rate around SV0.5h⁻¹ and around 85% of sucrose is transformed into isomaltulose. Then, the eluted syrup containing isomaltulose is desalinized with ion-exchange resin column, purified and concentrated, and then crystallized to give crystalline isomaltulose (See Shoichi SUZUKI and Yoshikazu NAKAJIMA "Property and use of palatinose®", Food *Chemicals, Special Issue-4* Shokuhin Kagaku Shinbunsha, Tokyo, Japan, Dec. 12, 1990, p165-171).

In those conventional processes, precipitation of supersaturated isomaltulose in the column or enzymatic reaction vessel tends to occur during the enzymatic reaction and the precipitated isomaltulose deteriorates recovery and reuse of the immobilized enzyme. In order to avoid supersaturation of isomaltulose during the enzymatic reaction, it is required to use relatively low concentration of aqueous sucrose, as low as 30-40 wt%, for the starting solution.

### SUMMARY OF THE INVENTION

An object of the present invention is to provide an efficient method for manufacturing crystalline isomaltulose with simple steps and facilities. Another object of the present invention to provide a method for manufacturing crystalline isomaltulose wherein the α-glucosyltransferase used therein is repeatedly used. According to the present invention, isomaltulose can be industrially manufactured with lower cost.

Further object of the present invention is to provide an efficient method for manufacturing hydrogenated isomaltulose.

Accordingly, the present invention provides a method for manufacturing crystalline isomaltulose from sucrose, comprising the steps of;
1) contacting an α-glucosyltransferase enzyme to aqueous sucrose solution or slurry under the condition wherein the α-glucosyltransferase enzyme is active; in which said condition is maintained after the concentration of isomaltulose in the reaction mixture reaches the point at which crystals are formed, and
2) separating the reaction mixture into crystalline isomaltulose and remaining syrup.

The present invention also provides the method as above further comprising the steps of:
3) adding sucrose to the remaining syrup to give reaction mixture,
4) placing the reaction mixture under the condition wherein the α-glucosyltransferase enzyme is active; in which said condition is maintained after the concentration of isomaltulose reaches the point at which crystals are formed,
5) separating the crystalline isomaltulose and remaining syrup, and
6) conducting the steps 3)-5) repeatedly.

The present invention further provides a method for manufacturing hydrogenated isomaltulose from the crystalline isomaltulose obtained by the instant method by dissolving the crystalline isomaltulose in water, and hydrogenating isomaltulose in the presence of a catalyst to give hydrogenated isomaltulose. Namely, the present invention provides a method for manufacturing hydrogenated isomaltulose from sucrose comprising the steps of:
1) contacting an α-glucosyltransferase enzyme to aqueous sucrose solution or slurry under the condition wherein the α-glucosyltransferase enzyme is active; in which said condition is maintained after the concentration of isomaltulose in the reaction mixture reaches the point at which crystals are formed,
2) separating the crystalline isomaltulose and remaining syrup,
   a) dissolving the crystalline isomaltulose in water, and
   b) hydrogenating isomaltulose in the presence of a catalyst to give hydrogenated isomaltulose.

   According to the method for manufacturing hydrogenated isomaltulose, the method of the present invention may further comprise the steps of;
3) adding sucrose to the remaining syrup to give reaction mixture,
4) placing the reaction mixture under the condition wherein the α-glucosyltransferase enzyme is active; in which said condition is maintained after the concentration of isomaltulose reaches the point at which crystals are formed,
5) separating the crystalline isomaltulose and remaining syrup, and
6) conducting the steps 3)-5) repeatedly after the step 2) and before the step a) of the above method.

According to the present invention, the enzymatic reaction and crystallization are carried out simultaneously in a same reaction vessel. In addition, the enzyme can be used repeatedly.

### BRIEF DESCRIPTION OF THE DRAWINGS

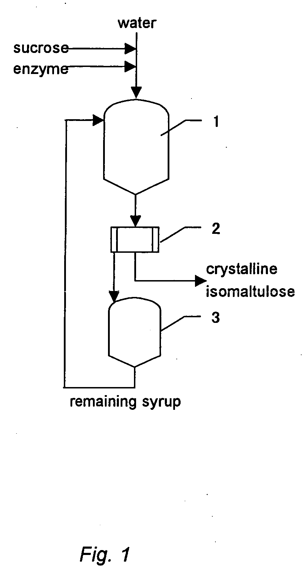

Fig. 1 shows the process flowchart of Example 1.

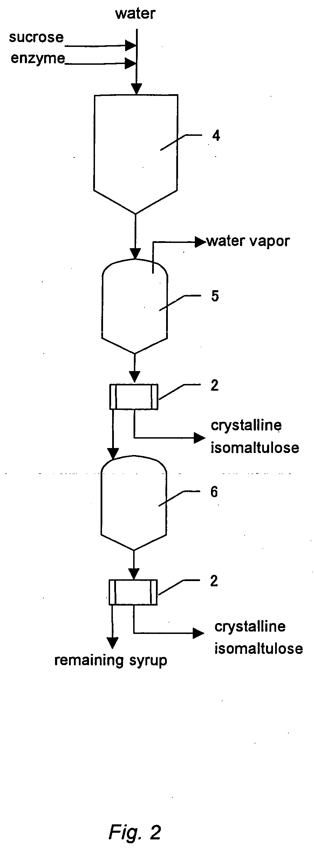

Fig. 2 shows the process flowchart of Comparative Example 1.

The symbols represents as follows:
1: reaction and crystallization vessel, 2: centrifuging equipment, 3: retention vessel, 4: reaction vessel, 5: concentration and crystallization vessel, 6: cooling and crystallization vessel.

### DETAILED DESCRIPTION

According to the present invention, sucrose used in the invention may be those of any sugar product available on the market irrespective of their purity. However, highly purified sucrose such as granulated sugar is preferable for manufacturing crystalline isomaltulose with higher purity.

According to the present invention, "solution" means a liquid mixture of water and component(s) wherein the concentration of the component is up to the saturated concentration. "Slurry" means a liquid-solid mixture of water and component (s) wherein the amount of the component(s) in the slurry is more than that provides the saturated concentration. "Supersaturated" or "supersaturation" means the situation in the liquid mixture wherein more than saturated concentration of component(s) is dissolved in water. "Sugar derivatives" represents saccharides such as sucrose, trehalulose, isomaltulose and others. "Syrup" represents a solution of sugar derivatives. In the context of the present invention, solution, slurry and syrup may contain the enzyme.

The starting aqueous sucrose solution or slurry may comprise sucrose in an amount of 50-90 wt%, preferably 60-80wt% on a dry weight basis. By using high concentration sucrose solution or slurry, α-glucosyltransferase becomes more temperature stable. When the concentration of the starting sucrose solution is less than 50 wt%, the concentration of the isomaltulose in the reaction mixture can hardly reach the point at which crystals are formed even if the enzymatic reaction is carried out under the optimized condition.

According to the present invention, the enzymatic reaction and crystallization are carried out in a same reaction vessel. Conventional reaction vessels or crystallization vessels such as those made of stainless-steel, FRP or glass may be employed for the present invention. Especially, reaction/crystallization vessels equipped with facilities for warm keeping and/or agitating may preferably be employed.

The α-glucosyltransferase used in the present invention may be any of those can transform or convert sucrose into isomaltulose. Said enzymes are well known in the art and examples may include known and unknown α-glucosyltransferases produced by various microorganisms such as of the genus *Serratia, Klebsiella,* Pseudomonas, *Protaminobacter, Elvinia* and *Agrobacterium.*

The enzyme may be subjected to the step of contacting with sucrose in the form of cell-free extract of the enzyme producing cells, homogenized cells or living cells. Cell free extract enzyme is preferably used since it is easy to be recovered from the reaction mixture after the enzymatic reaction is completed.

The condition wherein the α-glucosyltransferase is active is not limited as long as the enzyme is active under said condition. Accordingly, the condition may vary depending on properties of the enzyme used. For example, in case of α-glucosyltransferase obtained from a microorganism of genus *Serratia* is used, the preferable amount of the enzyme may be 1-100U per 1g of starting sucrose, and the reaction may preferably be carried out at 30-70°C, pH 5-7.

Under the condition, sucrose is enzymatically converted to isomaltulose, and the isomaltulose concentration in the reaction mixture increases as the enzymatic reaction progresses. When the isomaltulose concentration reaches the point of supersaturation, crystallization of isomaltulose occurs. Since the condition of the reaction mixture is maintained after crystallization of isomaltulose occurs, both of the enzymatic conversion of sucrose and the crystallization of isomaltulose proceed simultaneously in the same reaction mixture. Said condition is maintained until the enzymatic reaction is completed.

In order to effectively initiate crystallization of isomaltulose, isomaltulose seed crystals may preferably be added into the reaction mixture. The isomaltulose seed crystals used in the present invention may be any size as long as they can easily be dispersed in the gently agitated reaction mixture. In a typical embodiment, seed crystals having average particle size of 50-300µm are preferably used. The amount of the seed crystals to be added into the reaction mixture may vary dependent on their size and preferably be 0.1-10wt% of the total amount of the reaction mixture. The seed crystals may preferably be added to the reaction mixture after the isomaltulose concentration reaches the point of saturation.

The enzymatic reaction and the simultaneous crystallization may be continued, or the condition of the reaction mixture may be maintained until most of sucrose in the reaction mixture is consumed. In a typical embodiment, the sucrose concentration at the end of the enzymatic reaction may be less than 1.0 wt%. The time period of the enzymatic reaction/crystallization step may vary dependent on the condition of the reaction such as temperature, pH and the like, and the amount of sucrose, enzyme and seed crystals. In a typical embodiment, the step may be continued no more than 30 hours.

After the enzymatic reaction/crystallization step is completed, the resulting reaction mixture contains precipitated crystalline isomaltulose and syrup comprising unreacted sucrose, which may be less than 1.0 wt%, the active enzyme, isomaltulose and trehalulose. According to the present invention, next is the step of separating the crystalline isomaltulose and the remaining syrup. This step may be carried out by means of any known facilities for separating solid matter and liquid. For example, filtering facilities, such as leaf filter and filter press, and centrifugal separator may be employed and centrifugal separator is preferable for manufacturing highly purified isomaltulose efficiently. Centrifugal separator such as bucket type centrifugal separator may preferably be employed. The resulting reaction mixture containing crystalline isomaltulose may be centrifuged at 500-1500xG for 3-5 minutes to separate isomaltulose from the syrup.

The remaining syrup comprises active α-glucosyltransferase enzyme and accordingly, by adding further sucrose to the remaining syrup and placing the same under the condition wherein the enzyme is active, the added sucrose is enzymatically converted to isomaltulose.

Accordingly, the embodiment which comprises the steps of adding sucrose to the remaining syrup after the crystalline isomaltulose is removed, placing the same under the enzymatic reaction condition until the enzymatic reaction is completed and separating the crystalline isomaltulose and the remaining syrup is also covered by the present invention.

The amount of sucrose to be added to the remaining liquid may be up to the amount of the crystalline isomaltulose removed from the syrup. If desired, isomaltulose seed crystals may be added to the reaction mixture to efficiently initiate crystallization. Thus obtained reaction mixture may be placed under the condition wherein the α-glucosyltransferase is active. Said condition may be the same as above described condition.

Since the remaining syrup comprises saturated isomaltulose and active α-glucosyltransferase, crystallization of isomaltulose occurs within a short time after restart the enzymatic reaction. After the enzymatic reaction is completed, the precipitated crystalline isomaltulose is separated from the syrup. Those steps may also be conducted in the same manner as above.

In this embodiment, the enzymatic reaction/crystallization and separation steps are conducted repeatedly. Since the enzyme in the remaining syrup is active, the repeating cycle is not specifically limited. However, the trehalulose content in the remaining syrup, and also in the reaction mixture increases as the repeat number increases. The increased trehalulose may deteriorates crystal growth of isomaltulose. For the efficient manufacture of crystalline isomaltulose, the steps may preferably be completed when the ratio of isomaltulose to the total sugar derivatives in the remaining syrup becomes around 25wt%. After completed the repeating cycles, if desired, the final remaining syrup may be subjected to a known crystallization step such as cooling or concentrating to isolate the isomaltulose from the syrup so that total yield of isomaltulose is increased.

The isolated crystalline isomaltulose may be collected together and dried to give powdery product with good fluidity. Drying isomaltulose may be conducted by means of any known facility such as vacuum-ribbon type, fluid-bed type, shake type, rotary type and shake type and compartment tray type dryers. The drying may be carried out at any temperature so long as the crystal is not melted, and preferably, at 30-100°C and more preferably, at 60-90°C.

In another embodiment of the present invention, a method for manufacturing hydrogenated isomaltulose is also provided. In this embodiment, crystalline isomaltulose obtained by the above described method is hydrogenated. The hydrogenation may be carried out by means of any method known to the art. Those methods are well known to the art. For example, the crystalline isomaltulose is dissolved in water and hydrogenated in the presence of a reduction catalyst such as Raney-nickel at high temperature and pressure to give hydrogenated isomaltulose.

The present invention is further illustrated by following examples.

### EXAMPLE 1

### 1st Cycle

350g of water and 840g of granulated sugar were introduced into 2L-separable flask, which is kept over water bath at 50°C, and the mixture was agitated with a four-blade turbine (ϕ60mm, 60rpm) to give 70wt% sucrose solution. 10 g of cell free extract of *Serratia* containing 6720U of α-glucosyctransferase enzyme was added there to to start the enzymatic reaction. The reaction mixture was kept pH 6-7 by means of 0.1% aqueous sodium hydroxide. Five hours after the enzyme was added, 6g of crystalline isomaltulose having 100µm of average particle size was added as seed crystals to initiate crystallization. Twenty eight hours after the enzyme was added, the reaction mixture was slurry comprising 0.1wt% of sucrose. The obtained slurry was loaded into the centrifugal dehydrator equipped with nylon mesh filter (opening 250µm) and centrifuged for 10 minutes at 890xG to separate the syrup and the crystalline isomaltulose. Accordingly, crystalline isomaltulose 390g (solid content 93.5wt%; purity 98.6%) was obtained. The amount of the remaining syrup was 710g which comprised 57.3 wt% of sugar derivatives, and 71.2wt% of the sugar derivatives was isomaltulose (on a dry weight basis). 2nd Cycle

350g of granulated sugar and 650g of remaining syrup obtained in the 1st cycle were introduced into the 2L-separable flask and 5g of the seed crystals was added thereto. In the same manner as the 1st cycle, the enzymatic reaction and crystallization were conducted.

Twenty two hours after the start of the 2nd cycle, agitation was stopped and the reaction was completed. The sucrose content in the reaction mixture was 0.1wt%. 900g of thus obtained slurry was centrifuged in the same manner as the 1st cycle and 321g of crystalline isomaltulose (solid content 92.7wt%; purity 98.1%) was obtained. The remaining syrup was 579g, which contained 61.1 wt% of sugar derivatives, and 63.0wt% of the sugar derivatives was isomaltulose (on a dry weight basis).

### Comparative Example 1

### Enzymatic reaction:

1193.7g of water and 800g of granulated sugar were introduced into a 2L-separable flask, the flask was kept over water bath at 40°C while the mixture was agitated with a magnetic stirrer to give 40wt% sucrose solution. 6.3g of the same cell free extract as used in Example 1 was added thereto to start the enzymatic reaction. During the reaction, the reaction mixture was maintained pH 6-7 by means of 0.1% aqueous sodium hydroxide.

Thirty hours after the enzyme was added, the reaction was completed by stopping agitation. At the time, reaction mixture was liquid state solution comprising 82.0 wt% of isomaltulose and 0.2wt% of sucrose on a dry weight basis.

### 1st Crystallization: by means of heating under reduced pressure

1700 g of thus obtained reaction mixture was concentrated until thick syrup whose isomaltulose concentration was 56.7 wt% was provided under ambient pressure. 6g of the same isomaltulose seed crystals as used in Example 1 were added to 1200g of the thick syrup in 2L-separable flask. The flask was kept over the water bath at 60°C to keep the temperature of the mixture around 50°C and the mixture was agitated with four-blade turbine (ϕ60mm, 60rpm) under reduced pressure (75 Torr) so that water is evaporated. 250g of water was removed. Five hours after the seed crystals were added, agitation was stopped and crystallization was terminated. The obtained slurry 900g was centrifuged in the same manner as Example 1 to give 320g of crystalline isomaltulose (solid content 93.8wt%; purity 98.5%). The remaining syrup was 580g containing 59.6wt% of sugar derivatives and 68.0wt% of the sugar derivatives was isomaltulose.

### 2nd Crystallization: by means of cooling

400g of the remaining syrup obtained by the 1st crystallization step and 2g of the isomaltulose seed crystals were introduced into 2L-separable flask and the flask was kept over water bath at 50°C with agitating the mixture. The temperature of the water bath was gradually reduced at the rate of 1°C/min over 20 hours until the temperature is reduced to 30°C. Then, the mixture was stirred for more 5 hours at the same temperature. After the crystallization was completed by stopping agitation, 300g of the obtained slurry was centrifuged in the same manner as example 1 to give 57g of crystalline isomaltulose (solid content 93.6%, purity 98.4%). The remaining syrup was 243g containing 51.9wt% of sugar derivatives and 55.5 wt% of sugar derivatives was isomaltulose.

Conversion ratio from sucrose to isomaltulose, amount of the consumed sucrose, yield of crystalline isomaltulose, total amount of crystalline isomaltulose and energy consumption for evaporating water are calculated based on results of Example 1 and Comparative example 1 respectively, assuming no material was adhered to the reaction vessel, filter and the like.

**TABLE 1**

| | Example 1 | Comparative Ex.1 |
|---|---|---|
| total amount of starting reaction mixture . | 1200g | 1200g |
| amount of the added enzyme | 5.3U/1g sucrose | 5.3U/1g sucrose |
| temperature range | 50°C(constant) | 50-30°C |
| conversion ratio from sucrose to isomaltulose | 85.1% | 82.0% |
| total time | 50 hours | 60 hours |
| amount of total sucrose consumed | 1260g | 680g |
| yield of crystalline isomaltulose | 62.5% | 61.8% |
| total amount of obtained crystalline isomaltulose ¹⁾ | 788g | 420g |
| energy consumption for evaporating water ²⁾ (amount of evaporated water) | Okcal (0g) | 405kcal (750g) |

| | | |
|---|---|---|
| 1) Solid weight of obtained crystalline isomaltulose having a purity of 98% or more (the amount of the added seed crystals was deducted) | | |
| 2) The energy consumed for evaporating water in the crystallization step(kcal)=amount of the evaporated water(kg) × 540(kcal/kg) | | |

As is appeared from the table, the method of the present invention can produce more isomaltulose in a shorter time than the conventional method. In addition, the process of the present invention need not to evaporate water for crystallization and does not consume the energy required for evaporation.

## Claims

1. A method for manufacturing crystalline isomaltulose from sucrose, comprising the steps of;
1) contacting an α-glucosyltransferase enzyme to aqueous sucrose solution or slurry under the condition wherein the α-glucosyltransferase enzyme is active; in which said condition is maintained after the concentration of isomaltulose in the reaction mixture reaches the point at which crystals are formed, and
2) separating the reaction mixture into crystalline isomaltulose and remaining syrup.

2. The method of Claim 1, wherein the starting aqueous sucrose solution or slurry comprises 50-90 wt% of sucrose on a dry weight basis.

3. The method of Claim 1 or 2, further comprising the step of adding isomaltulose seed crystals to the reaction mixture to initiate crystallization of isomaltulose.

4. The method of any of Claims 1-3, further comprising the steps of;
3) adding sucrose to the remaining syrup to give reaction mixture,
4) placing the reaction mixture under the condition wherein the α-glucosyltransferase enzyme is active; in which said condition is maintained after the concentration of isomaltulose reaches the point at which crystals are formed,
5) separating the reaction mixture into crystalline isomaltulose and remaining syrup, and
6) conducting the steps 3)-5) repeatedly.

5. The method of Claim 4, wherein the steps 3)-5)are conducted repeatedly until the ratio of isomaltulose to the total sugar derivatives in the remaining syrup becomes less than 25wt% on a dry weight basis.

6. A method for manufacturing hydrogenated isomaltulose, comprising the steps of;
1) contacting an α-glucosyltransferase enzyme to aqueous sucrose solution or slurry under the condition wherein the α-glucosyltransferase enzyme is active; in which said condition is maintained after the concentration of isomaltulose in the reaction mixture reaches the point at which crystals are formed,
2) separating the crystalline isomaltulose and remaining syrup,
a) dissolving the crystalline isomaltulose in water, and
b) hydrogenating isomaltulose in the presence of a catalyst to give hydrogenated isomaltulose.

7. The method of Claim 6, wherein the starting aqueous sucrose solution or slurry comprises 50-90 wt% of sucrose on a dry weight basis.

8. The method of Claim 6 or 7, further comprising the step of adding isomaltulose seed crystals to the reaction mixture to initiate crystallization of isomaltulose.

9. The method of any of Claims 6-8, further comprising the steps of;
3) adding sucrose to the remaining syrup to give reaction mixture,
4) keeping the reaction mixture under the condition wherein the α-glucosyltransferase enzyme is active; in which said condition is kept after the concentration of isomaltulose reaches the point at which crystals are formed, and
5) separating the crystalline isomaltulose and remaining syrup, and
6) conducting the steps 3)-5) repeatedly between after the step 2) and before the step a).

10. The method of Claim 9, wherein the steps 3)-5)are conducted repeatedly until the ratio of isomaltulose to the total sugar derivatives in the remaining syrup becomes less than 25wt% on a dry weight basis.
